# FASCICULE DE BREVET EUROPEEN

(11) **EP 1 178 787 B1**
(45) Date de publication et mention de la délivrance du brevet: **30.07.2003**
(21) Numéro de dépôt: 00931319.8
(22) Date de dépôt: 19.05.2000
(51) Int. Cl.: A61K 31/05, A61K 9/00

(54) **COMPOSITIONS PHARMACEUTIQUES, DESTINEES A L'ADMINISTRATION PAR VOIE ORALE DE PHLOROGLUCINOL ET LEUR PREPARATION**
PHARMAZEUTISCHE ZUSAMMENSETZUNGEN, BESTIMMT ZUR ORALEN VERABREICHUNG VON PHLOROGLUCINOL SOWIE DEREN HERSTELLUNG
PHARMACEUTICAL COMPOSITIONS FOR ORAL ADMINISTRATION OF PHLOROGLUCINOL AND PREPARATION THEREOF

(30) Priorité: 19.05.1999 FR 9906325
(43) Date de publication de la demande: 13.02.2002
(73) Titulaire: Promindus (Actions Promotionnelles dans l'Industrie et le Commerce), 21000 Casablanca (MA)
(72) Inventeur: BENNIS, Abderrahim, 21000 Casablanca (MA); SERRANO, Jean-Jacques, F-34090 Montpellier (FR); BENNIS, Farid, 21000 Casablanca (MA)
(74) Mandataire: Le Roux, Martine
(86) Numéro de dépôt international: FR0001365
(87) Numéro de publication internationale: WO00071110

(56) Documents cités:
- EP-A- 0 643 962
- WO-A-92/02209
- WO-A-97/17064
- FR-A- 1 569 456
- FR-A- 2 722 408
- FR-M- 784

## Description

La présente invention a pour objet des compositions pharmaceutiques, destinées à l'administration par voie orale de phloroglucinol (1,3,5-trihydroxy benzène) et leur préparation. Lesdites compositions, originales, sont intéressantes, en ce qu'en leur sein, l'activité antispasmodique du phloroglucinol (activité antispasmodique sur les fibres musculaires lisses) est potentialisée.

Ladite activité antispasmodique dudit phloroglucinol est connue depuis 1961 (on peut notamment se référer à Debray et al., THERAPIE, 1961, 16, pages 978 à 990 et Cahen et al., THERAPIE, 1962, page 17). Ainsi, le phloroglucinol est-il utilisé dans le traitement des manifestations spasmodiques et douloureuses des voies urinaires, des voies biliaires, des voies digestives et de la sphère gynécologique. Il est, à ce jour, administré, par voie orale, sous forme de comprimés ou de lyophilisats, par voie rectale, sous forme de suppositoires, ou par voie injectable (I.M. ou I.V.). Pour l'administration par voie orale, les lyophilisats sont généralement préférés dans la mesure où ils exhibent une biodisponibilité plus rapide et plus complète que les comprimés. Lesdits lyophilisats sont plus rapidement actifs. La posologie usuelle du phloroglucinol, par voie orale, est généralement de 160 mg, par prise de deux comprimés ou lyophilisats.

Dans un tel contexte, la Demanderesse propose présentement une forme galénique originale pour l'administration, par voie orale, dudit phloroglucinol. Ladite forme galénique originale peut se décliner selon de nombreuses variantes. Elle peut être *per se* nouvelle (voir, par exemple, les comprimés, granulés ou poudres effervescents décrits plus avant dans le présent texte) ou elle peut consister en une forme galénique classique modifiée (voir, par exemple, les comprimés ou lyophilisats décrits plus avant dans le présent texte). Quelle que soit sa forme de réalisation, ladite forme galénique est, de façon caractéristique, tamponnée à un pH compris entre 3 et 7.

Ainsi, selon son principal objet, la présente invention concerne-t-elle, des compositions pharmaceutiques destinées à l'administration par voie orale de phloroglucinol, caractérisées en ce que, liquides, elles renferment un système les tamponnant à un pH compris entre 3 et 7 ou en ce que, solides, elles renferment un système susceptible d'exercer, lors de leur mise en milieu aqueux, un effet tampon entre pH 3 et pH 7.

La composition des compositions pharmaceutiques de l'invention est, de façon caractéristique, telle qu'elle exerce un effet tampon, dans la zone de pH énoncée ci-dessus, zone de pH, délimitée par lesdites valeurs pH 3 et pH 7, qui inclut lesdites valeurs pH 3 et pH 7. L'exercice dudit effet tampon dans ladite zone de pH (3 ≤ pH ≤ 7) est bien évidemment compatible avec la stabilité du principe actif intervenant : le phloroglucinol (ce composé, oxydable en milieu alcalin, ne doit pas en effet être exposé à des pH > 7) ; il permet de diminuer l'acidité gastrique et de façon tout à fait surprenante il potentialise l'activité antispasmodique dudit phloroglucinol. Ainsi, des comprimés effervescents tamponnés au sens de l'invention se sont-ils révélés quasi aussi efficaces qu'une injection intramusculaire et des lyophilisats oraux tamponnés au sens de l'invention se sont-ils aussi révélés plus efficaces que des lyophilisats oraux de l'art antérieur (non tamponnés).

Avantageusement, les compositions pharmaceutiques de l'invention sont tamponnées à un pH compris entre 4 et 6 (4 ≤ pH ≤ 6).

On a déjà vu ci-dessus que lesdites compositions pharmaceutiques, tamponnées au sens de l'invention, peuvent exister sous diverses formes. Elles peuvent notamment se présenter sous des formes liquides (directement tamponnées à un pH adéquat) telles des solutés, des suspensions, des sirops ou sous des formes solides (qui développeront l'effet tampon, lors de leur prise, dans un liquide, généralement de l'eau, ou suite à leur prise, dans l'estomac) telles des comprimés (effervescents ou non, avantageusement effervescents, voir plus loin), des gélules, des poudres (effervescentes ou non, avantageusement effervescentes, voir plus loin), des granulés (effervescents ou non, avantageusement effervescents, voir plus loin), des lyophilisats. Cette liste n'est pas exhaustive.

L'homme du métier, spécialiste de la galénique, saura, en tout état de cause, formuler le phloroglucinol, notamment sous l'une ou l'autre des formes unitaires listées ci-dessus, avec un système adéquat, responsable de l'effet tampon recherché. De telles formes unitaires (comprimés par exemple, et notamment comprimés classiques, comprimés à double noyau, comprimés effervescents) constituent évidemment, avantageusement, l'essentiel des compositions pharmaceutiques de l'invention. Il ne saurait toutefois être totalement exclu du cadre de l'invention, les compositions pharmaceutiques à au moins deux composants séparés (un composant renfermant au moins le principe actif, d'une part, un autre composant renfermant au moins le système générateur de l'effet tampon désiré, d'autre part) ; composants séparés à administrer conjointement.

Dans le cadre d'un mode de réalisation préféré de l'invention, ledit système, responsable de l'effet tampon, comprend l'association d'au moins un acide organique et/ou d'au moins un sel d'acide organique avec au moins une base forte et/ou au moins un sel d'une base forte.

Dans le cadre de ce mode de réalisation préféré, ledit acide organique est avantageusement choisi parmi les acides citrique, tartrique, malique, lactique, acétique, glutarique, benzoïque et adipique ou/et ladite base intervient sous la forme de bicarbonate de sodium, de carbonate de sodium, de carbonate de calcium, de carbonate de magnésium, d'hydroxyde de sodium, d'hydroxyde de potassium, de bicarbonate de potassium ou de carbonate de potassium.

De façon particulièrement avantageuse, les compositions pharmaceutiques de l'invention consistent en des formes galéniques solides effervescentes ; elles se présentent notamment sous la forme de comprimés effervescents, de granulés effervescents ou de poudres effervescentes. Dans le cadre de cette variante avantageuse, le même système est généralement et opportunément responsable de l'effet tampon recherché et de l'effervescence.

Selon l'invention, les comprimés effervescents de phloroglucinol sont tout particulièrement préférés. De tels comprimés se sont révélés plus efficaces que les lyophilisats oraux de l'art antérieur et de surcroît, leur fabrication est moins coûteuse que celle desdits lyophilisats oraux.

De tels comprimés sont susceptibles de renfermer les associations acide(s) organique(s) et/ou sel(s) d'acide organique/base(s) forte(s) et/ou sel(s) de base forte définies ci-dessus. Avantageusement, ils renferment la combinaison acide citrique/bicarbonate de sodium.

Il est donc du mérite des inventeurs d'avoir établi que l'effet tampon précisé ci-dessus potentialise l'activité antispasmodique du phloroglucinol et de proposer de nouvelles formes galéniques dudit phloroglucinol à activité antisspasmodique potentialisée, notamment des formes effervescentes.

La préparation des compositions pharmaceutiques de l'invention, telles que décrites ci-dessus, constitue le second objet de ladite invention. Ladite préparation est une préparation de forme galénique tamponnée. De façon caractéristique, elle comprend la formulation du phloroglucinol, sous forme liquide, avec un système tamponnant ladite forme liquide à un pH compris entre 3 et 7 (avantageusement, entre 4 et 6) ou, sous forme solide, avec un système susceptible d'exercer, lors de la mise en milieu aqueux de ladite forme solide, un effet tampon entre pH 3 et pH 7 (avantageusement, entre pH 4 et pH 6).

On a déjà indiqué que ladite préparation ne devrait pas soulever de quelconque problème à un homme du métier spécialiste de galénique.

A toutes fins utiles, on se propose de préciser ci-après, à titre purement illustratif, un mode opératoire avantageux de préparation de comprimés effervescents de phloroglucinol.

Le principe actif, phloroglucinol dihydraté, est tout d'abord mélangé avec le système responsable, à la fois, de l'effervescence et de l'effet tampon recherché : acide citrique + bicarbonate de sodium. Audit mélange, il est avantageusement ajouté de faibles quantités d'additifs, type lubrifiant (benzoate de sodium, par exemple) et/ou conservateur et/ou édulcorant (saccharose sodique, par exemple) ...

Le mélange de poudres résultant est tamisé puis granulé avec un solvant hydroalcoolique. Les granulés obtenus sont, successivement, séchés, calibrés. On met alors en oeuvre un contrôle de leur taux d'humidité résiduelle. Ils sont finalement lubrifiés puis comprimés pour être agglomérés sous la forme de comprimés. Lesdits comprimés sont ensuite conditionnés dans leur emballage primaire.

Ce procédé de fabrication de comprimés effervescents n'est pas *per se* nouveau. Il est original en ce qu'il est mis en oeuvre avec du phloroglucinol,

A titre purement illustratif, on peut encore préciser ci-après la composition pondérale d'un comprimé effervescent de l'invention :

| | |
|---|---|
| Phloroglucinol (dihydrate) | 80 ,0 mg |
| Acide citrique | 297,2 mg |
| Bicarbonate de sodium | 362,6 mg |
| Benzoate de sodium | 15,2 mg. |

Dissous dans un verre d'eau, un tel comprimé génère une solution tamponnée à pH = 4,5.

On se propose enfin d'illustrer l'intérêt de la présente invention en présentant ci-après des résultats comparatifs de tests pharmacologiques.

Au cours desdits tests, l'activité antispasmodique de différentes formes galéniques du phloroglucinol a été évaluée à l'aide du test de SIEGMUND. On rappelle ci-après brièvement, le principe de ce test, familier à l'homme du métier.

Le syndrome douloureux provoqué par l'injection intrapéritonéale de 0,25 ml d'une solution de phénylbenzoquinone chez la souris se caractérise par des mouvements d'étirement des pattes postérieures et de torsion de la musculature dorso-abdominale, que l'on comptabilise pendant un laps de temps de 30 min, à partir de l'expiration des 15 min qui suivent l'administration de ladite phénylbenzoquinone. Un effet antispasmodique se manifeste par une réduction du nombre de ces mouvements. Pour chaque test, la substance à l'étude est administrée, par voie intragastrique ou autre, 30 min avant l'administration de ladite phénylbenzoquinone.
. Une première étude a été réalisée sur trois lots de souris.
   Un comprimé effervescent de l'invention, dosé à 80 mg de phloroglucinol, a été dissous dans de l'eau distillée de façon à ce que la dose de 100 mg/kg ait été administrée sous un volume de 20 ml/kg, à l'aide d'une sonde oesophagienne (Lot A de l'invention).
   Les témoins (Lot B) ont reçu de l'eau distillée sous un même volume.
   On a préparé une solution aqueuse au même dosage à partir de lyophilisats oraux (Lyoc) de l'art antérieur. Elle a été administrée dans les mêmes conditions (Lot C).
   Les résultats obtenus ont été exprimés, en pourcentage de protection contre le spasme induit par la phénylbenzoquinone, par rapport aux témoins. Ils sont indiqués ci-après :
   Lot C : Lyoc : 28 % (non significatif par rapport aux témoins (Lot B))
   Lot A : Composé effervescent : 47 % (significatif à p > 0,001).

   Le comprimé effervescent exhibe une activité antispasmodique nettement supérieure à celle du lyophilisat oral.
. Dans des conditions similaires, évidemment comparatives, on a évalué ledit pourcentage d'inhibition du spasme, par rapport à un lot témoin, à différents dosages (40 mg/kg, 80 mg/kg et 160 mg/kg), du phloroglucinol(dihydraté) formulé :
   - sous forme de lyophilisat oral : LYOC (art antérieur),
   - sous forme de soluté injectable : I.M. (art antérieur),
   - sous forme de comprimé effervescent : EFFERV. (invention),
   - sous forme de lyophilisât oral tamponné : LYOC' (invention) ; dans ce quatrième cas, on a en fait mis en oeuvre un artifice. Un lyophilisat de l'art antérieur (LYOC) a été mis en solution dans de l'eau distillée et tamponné par de l'acide citrique et du bicarbonate de sodium de manière à obtenir un pH de 5 (LYOC').

Les résultats obtenus sont exprimés, comme ci-dessus, dans le tableau ci-après :

| | Pourcentage d'inhibition du spasme | | |
|---|---|---|---|
| | 40 mg/kg | 80 mg/kg | 160 mg/kg |
| LYOC | 6 | 24 | 34* |
| I.M. | 12 | 43*** | 59*** |
| EFFERV. | 20 | 43*** | 53*** |
| LYOC' | | | 45*** |

| | | | |
|---|---|---|---|
| * p = 0,05 | | | |
| *** p = 0,001 | | | |

L'analyse statistique réalisée entre LYOC et I.M. ou EFFERV. à 80 mg montre une différence hautement significative p = 0,001.

L'analyse statistique réalisée entre LYOC et I.M. ou EFFERV. à 160 mg montre une différence hautement significative p = 0,01.

L'analyse statistique réalisée entre I.M. et EFFERV. à 160 mg montre que la différence n'est pas significative.

L'analyse statistique réalisée entre LYOC et LYOC' à 160 mg montre une différence statistiquement significative p = 0,05.

L'analyse statistique réalisée entre EFFERV. et LYOC' à 160 mg montre une différence non significative.

A la considération des chiffres dudit tableau, on ne manque pas de saisir tout l'intérêt de la présente invention.

## Revendications

1. Compositions pharmaceutiques, destinées à l'administration par voie orale de phloroglucinol, **caractérisées en ce que**, liquides, elles renferment un système les tamponnant à un pH compris entre 3 et 7 ou **en ce que**, solides, elles renferment un système susceptible d'exercer, lors de leur mise en milieu aqueux, un effet tampon entre pH 3 et pH 7.

2. Compositions pharmaceutiques selon la revendication 1, **caractérisées en ce que** ledit pH tampon est compris entre 4 et 6.

3. Compositions pharmaceutiques selon l'une des revendications 1 ou 2, **caractérisées en ce qu'**elles se présentent sous la forme de solutés, de suspensions, de sirops ou sous la forme de comprimés, de gélules, de poudres, de granulés, de lyophilisats.

4. Compositions pharmaceutiques selon l'une quelconque des revendications 1 à 3, **caractérisées en ce que** ledit système, responsable de l'effet tampon, comprend l'association d'au moins un acide organique et/ou d'au moins un sel d'acide organique avec au moins une base forte et/ou au moins un sel d'une base forte.

5. Compositions pharmaceutiques selon la revendication 4, **caractérisées en ce que** ledit acide organique est choisi parmi les acides citrique, tartrique, malique, lactique, acétique, glutarique, benzoïque et adipique.

6. Compositions pharmaceutiques selon l'une des revendications 4 ou 5, **caractérisées en ce que** ladite base intervient sous la forme de bicarbonate de sodium, de carbonate de sodium, de carbonate de calcium, de carbonate de magnésium, d'hydroxyde de sodium, d'hydroxyde de potassium, de bicarbonate de potassium ou de carbonate de potassium.

7. Compositions pharmaceutiques selon l'une quelconque des revendications 1 à 6, **caractérisées en ce qu'**elles se présentent sous la forme de préparations galéniques solides effervescentes.

8. Compositions pharmaceutiques selon l'une quelconque des revendications 1 à 7, **caractérisées en ce qu'**elles se présentent sous la forme de comprimés effervescents.

9. Compositions pharmaceutiques selon l'une quelconque des revendications 1 à 7, **caractérisées en ce qu'**elles se présentent sous la forme de comprimés effervescents renfermant de l'acide citrique et du bicarbonate de sodium.

10. Procédé de préparation de compositions pharmaceutiques selon l'une quelconque des revendications précédentes, **caractérisé en ce qu'**il comprend la formulation du phloroglucinol, sous forme liquide, avec un système tamponnant ladite forme liquide à un pH compris entre 3 et 7 ou, sous forme solide, avec un système susceptible d'exercer, lors de la mise en milieu aqueux de ladite forme solide, un effet tampon entre pH 3 et pH 7.

## Patentansprüche

1. Pharmazeutische Zusammensetzungen für die orale Verabreichung von Phloroglucin, **dadurch gekennzeichnet, dass** sie im flüssigen Zustand ein Puffersystem bei einem pH-Wert zwischen 3 und 7 aufweisen oder im festen Zustand ein System enthalten, das bei ihrer Überführung in ein wässriges Medium eine Pufferwirkung zwischen pH 3 und pH 7 ausüben kann.

2. Pharmazeutische Zusammensetzungen nach Anspruch 1, **dadurch gekennzeichnet, dass** die pH-Pufferung zwischen 4 und 6 liegt.

3. Pharmazeutische Zusammensetzungen nach einem der Ansprüche 1 oder 2, **dadurch gekennzeichnet, dass** sie in Form von Lösungen, Suspensionen, Sirupen oder in Form von Tabletten, Kapseln, Pulvern, Granulaten, Lyophilisaten vorliegen.

4. Pharmazeutische Zusammensetzungen nach einem der Ansprüche 1 bis 3, **dadurch gekennzeichnet, dass** das System, das für die Pufferwirkung verantwortlich ist, eine Kombination von mindestens einer organischen Säure und/oder mindestens einem Salz einer organischen Säure mit mindestens einer starken Base und/oder mindestens einem Salz einer starken Base umfasst.

5. Pharmazeutische Zusammensetzungen nach Anspruch 4, **dadurch gekennzeichnet, dass** die organische Säure ausgewählt ist aus der Gruppe Citronensäure, Weinsäure, Apfelsäure, Milchsäure, Essigsäure, Glutarsäure, Benzoesäure und Adipinsäure.

6. Pharmazeutische Zusammensetzungen nach einem der Ansprüche 4 oder 5, **dadurch gekennzeichnet, dass** die Base in Form von Natriumbicarbonat, Natriumcarbonat, Calciumcarbonat, Magnesiumcarbonat, Natriumhydroxid, Kaliumhydroxid, Kaliumbicarbonat oder Kaliumcarbonat eingesetzt wird.

7. Pharmazeutische Zusammensetzungen nach einem der Ansprüche 1 bis 6, **dadurch gekennzeichnet, dass** sie in Form von festen schäumenden galenischen Präparaten vorliegen.

8. Pharmazeutische Zusammensetzungen nach einem der Ansprüche 1 bis 7, **dadurch gekennzeichnet, dass** sie in Form von Brausetabletten vorliegen.

9. Pharmazeutische Zusammensetzungen nach einem der Ansprüche 1 bis 7, **dadurch gekennzeichnet, dass** sie in Form von Brausetabletten vorliegen, die Citronensäure und Natriumbicarbonat enthalten.

10. Verfahren zur Herstellung der pharmazeutischen Zusammensetzungen nach einem der vorhergehenden Ansprüche, **dadurch gekennzeichnet, dass** es umfasst die Formulierung von Phloroglucin in flüssiger Form mit einem Puffersystem, das die flüssige Form auf einen pH-Wert zwischen 3 und 7 abpuffert, oder in fester Form mit einem System, das bei der Einführung der festen Form in ein wässriges Medium eine Pufferwirkung zwischen pH 3 und pH 7 ergeben kann.

## Claims

1. Pharmaceutical compositions for the oral administration of phloroglucinol, **characterized in that**, when liquid, they contain a system which buffers them to a pH of between 3 and 7, or **in that**, when solid, they contain a system which, when they are placed in an aqueous medium, is capable of exerting a buffer effect between pH 3 and pH 7.

2. The pharmaceutical compositions according to claim 1, **characterized in that** said buffer pH is between 4 and 6.

3. The pharmaceutical compositions according to claim 1 or 2, **characterized in that** they are presented in the form of solutions, suspensions or syrups or in the form of tablets, gelatin capsules, powders, granules or lyophilizates.

4. The pharmaceutical compositions according to any one of claims 1 to 3, **characterized in that** said system responsible for the buffer effect comprises at least one organic acid and/or at least one salt of an organic acid in association with at least one strong base and/or at least one salt of a strong base.

5. The pharmaceutical compositions according to claim 4, **characterized in that** said organic acid is selected from citric, tartaric, malic, lactic, acetic, glutaric, benzoic and adipic acids.

6. The pharmaceutical compositions according to claim 4 or 5, **characterized in that** said base takes the form of sodium bicarbonate, sodium carbonate, calcium carbonate, magnesium carbonate, sodium hydroxide, potassium hydroxide, potassium bicarbonate or potassium carbonate.

7. The pharmaceutical compositions according to any one of claims 1 to 6, **characterized in that** they are presented in the form of effervescent solid galenical preparations.

8. The pharmaceutical compositions according to any one of claims 1 to 7, **characterized in that** they are presented in the form of effervescent tablets.

9. The pharmaceutical compositions according to any one of claims 1 to 7, **characterized in that** they are presented in the form of effervescent tablets containing citric acid and sodium bicarbonate.

10. Process for the preparation of pharmaceutical compositions according to any one of the preceding claims, **characterized in that** it comprises formulating the phloroglucinol in the liquid form with a system which buffers said liquid form to a pH of between 3 and 7, or in the solid form with a system which, when said solid form is placed in an aqueous medium, is capable of exerting a buffer effect between pH 3 and pH 7.
